# EUROPEAN PATENT APPLICATION

(11) **EP 0 716 095 A1**
(43) Date of publication of application: **12.06.1996**
(21) Application number: 95119179.0
(22) Date of filing: 06.12.1995
(51) Int. Cl.: C07K 16/28, A61K 39/00, C12P 21/08, G01N 33/577

(54) **Method for quantitative determination of fas antigen**

(30) Priority: 08.12.1994 JP 330485/94
(71) Applicant: ORIENTAL YEAST CO., LTD., Tokyo (JP)
(72) Inventor: Okumura, Ko, Chiba-shi, Chiba-ken (JP); Yagita, Hideo, Tokyo (JP); Nakata, Motomi, Yokohama-shi, Kanagawa-ken (JP); Arai, Hideo, Kazo-shi, Saitama-ken (JP)
(74) Representative: Türk, Gille, Hrabal, Leifert

(57) **Abstract**

The present invention relates to a polyclonal antibody or a monoclonal antibody which is obtained by using, as the immunogen, a synthetic peptide having the following sequence (a part of which may be modified or deleted) or a soluble Fas antigen containing an intracellular region.
Cys-Asn-Ile-Thr-Ser-Asp-Ser-Gln-Asn-Ser-Asn-Phe-Arg-Asn-Glu-Ile-Gln-Ser-Leu-Val
These antibodies specifically recognize the intracellular region of a Fas antigen. By measuring a Fas antigen according to sandwich ELISA using any of these antibodies, it is possible to diagnose autoimmune diseases such as SLE, RA, etc.

## Description

### Detailed Description of the Invention:

### Industrial Field of the Invention:

The present invention relates to specific antibodies that recognize the intracellular region of a Fas antigen. The anti-Fas antibodies can specifically recognize even a soluble form Fas (soluble Fas) containing an intracellular region. Utilizing the property of the antibodies, therefore, we, the present inventors have succeeded in, for the first time, the establishment of an efficient and accurate assay system for such a soluble Fas according to an ELISA method.

According to the present invention, it is possible to clarify the mode of the existence of a soluble Fas, namely, to clarify as to whether the soluble Fas contains an intracellular region or has only an extracellular region (in the latter case, the soluble Fas is of the form as cut on the surface of a cell). By utilizing the present invention, therefore, it is extremely easy to diagnose autoimmune diseases such as SLE and RA from blood samples.

### Prior Art:

Fas is an apotosis signaling receptor molecule existing on the surfaces of many cells and is a protein comprising an intracellular region and an extracellular region. However, since only the antibody to the extracellular region of Fas has heretofore been developed, it has heretofore been impossible to selectively measure a soluble Fas containing an intracellular region.

Most recently, it has been clarified that a larger amount of soluble form Fas exists in serum samples collected from patients suffering from systemic lupus erythematosus (SLE) than that in serum samples from normal persons (see Science, Vol. 263, 25, March 1994, pp. 1759-1762). Given the situation, it has become necessary to accurately analyze only the soluble Fas containing an intracellular region while differentiating it from the soluble Fas in an extracellular region in order to diagnose autoimmune diseases including SLE.

### Problems to be Solved by the Invention:

The present invention has been attained in the current situation of the art. As has been mentioned hereinabove, only the antibody that recognizes Fas in an extracellular region has heretofore been developed. Therefore, in quantitatively determining the soluble form Fas that has been found this year by the use of the known antibody, it is impossible to clarify the mode of its existence as to whether the soluble form Fas contains an intracellular region or it has only an extracellular region. The present invention is to develop antibodies specific to the intracellular region (C-terminal) of Fas and to establish a combination of the C-terminal-specific antibody/antibodies and the known antibody applicable to the system of measuring a soluble Fas antigen, thereby clarifying the mode of the existence of the soluble Fas by the use of the thus-established combination.

### Brief Description of the Drawings:

Fig. 1 shows the titer of the anti-Fas polyclonal antibody of the present invention.
Fig. 2 shows the standard curve of ELISA conducted according to the present invention.
Fig. 3 shows the data of the soluble form Fas concentration in the sera collected from normal persons, SLE patients and RA patients, as measured by ELISA according to the present invention.

### Means for Solving the Problems:

We, the present inventors have studied from all angles in order to attain the object of the present invention as mentioned above. Specifically, we have synthesized a peptide comprising the C-terminal of the intracellular region of a Fas antigen and immunized animals with the peptide. As a result of the immunization, we have succeeded, for the first time, in the production of a polyclonal antibody that is specific to the C-terminal of the intracellular region of a Fas antigen in the immunized sera and also in the purification of the polyclonal antibody. In addition, we have confirmed for the first time that immunized cells that produce the C-terminal-specific antibody can be obtained by immunizing animals with the synthetic peptide, that the immunized cells form hybridomas after having been fused with myeloma cells and that a C-terminal-specific monoclonal antibody can be obtained by incubating the hybridomas.

Further, we, the present inventors have newly developed a sandwich ELISA method comprising the combination of the antibody/antibodies and an antibody that recognizes the extracellular region of Fas and have confirmed that only the soluble form Fas containing an intracellular region, which is in various samples such as sera, etc., can be selectively measured according to this method. As a result of these, we have succeeded in the establishment of an assay system specific to the soluble form Fas.

In addition, we, the present inventors have confirmed that the assay method now established by us have various advantages in that only a soluble form Fas containing an intracellular region, which is in the serum of a patient suffering from any of autoimmune diseases such as SLE and RA (rheumatoid arthritis), can be measured accurately according to the assay method and that the assay method can be utilized in diagnosis of such diseases. On the basis of these findings, we have further studied and, as a result, have completed the present invention. The present invention is described in detail hereinunder.

As the immunogen to be employed for forming the antibodies of the present invention, used is a whole molecule of a Fas antigen (that is, a soluble Fas antigen containing an intracellular region) or the C-terminal of the intracellular region of such a Fas antigen which is synthesized by the use of a peptide synthesizer. As one example of such synthetic peptides, mentioned is a peptide having the amino acid sequence mentioned below, which is comprised of from 1 to 20 amino acids.
Cys-Asn-Ile-Thr-Ser-Asp-Ser-Gln-Asn-Ser-Asn-Phe-Arg-Asn-Glu-Ile-Gln-Ser-Leu-Val
A part of the amino acids constituting the above-mentioned synthetic peptide can be modified into other amino acid(s) or can be eliminated to form modified peptides, which can also be used in the present invention as the immunogen like the synthetic peptide.

Where the synthetic peptides are used as the immunogens in the present invention, animals are immunized with any of the immunogens while using a Freund's adjuvant or other various adjuvants and/or bonding to the immunogen any of various carriers such as bovine thyroglobulin, BSA (bovine serum albumin), KLH (keyhole limpet hemocyanine) and the like for the purpose of promoting the immune response in the animals

Various animals such as rabbits, rats, mice, goats and the like are immunized necessary times with any of these antigens, and anti-sera are collected therefrom by ordinary methods. To purify the intended synthetic peptide-specific antibody from the anti-sera, employable are ordinary antibody-purifying methods of salting out with ammonium sulfate, affinity purification, ion-exchange column chromatography, molecular sieve column chromatography (gel permeation), protein A column chromatography, etc.

For affinity purification (by antigen-fixed column chromatography) using the synthetic peptide, the peptide is fixed to the column, the anti-serum is charged into the column thereby making the specific antibody adsorbed to the column, and then the specific antibody is eluted by the use of an eluent and is collected. In this manner, a high-purity anti-Fas polyclonal antibody can be obtained. In order to increase the purity of the antibody to be obtained, the purification means are combined or the purification is repeated.

A monoclonal antibody of the present invention can be obtained in the manner mentioned below.

First, animals such as male BALB/c mice are immunized with any of the above-mentioned synthetic peptides (including modified peptides) or with the whole molecule of a Fas antigen, as the immunogen, until the thus-immunized animals have a sufficient antibody value, and thereafter the immunized cells of the spleen, the lymphonodus are taken out from the animals in a germ-free condition. These cells are used for cell fusion.

For example, the spleen is taken out from the immunized mouse in a germ-free condition, and thereafter the spleen cells are separated from this and fused with mouse myeloma cells in the presence of a suitable cell fusion promoter. As the myeloma cells to be used for the cell fusion, mentioned are P3-Ag8-gamma, p3-X63-Ag8, P3-X63-Ag8-U1, NSI-Ag4/1, X63-Ag8-6, 5, 3, SP2/0-Ag14, MPC11-45.6TG1.7, S194/5XX0, BU, 1, etc.

As the preferred cell fusion promoter for use in the present invention, for example, mentioned is a polyethylene glycol having a molecular weight of from 1000 to 6000.

Electric pulse may also be applied to the cell fusion for the present invention.

Since the myeloma cells to be subjected to the cell fusion are of a 8-azaguanine-resistant strain and do not have hypoxanthine-guanine-phosphoribosyl transferase that is necessary for the salvage pathway for biosynthesis of nucleotides, they cannot produce nucleotides in a HAT medium (or a medium comprising hypoxanthine, aminopterin and thymidine) and therefore cannot live therein.

Accordingly, after the cell fusion, the cells are incubated in a HAT medium for a predetermined period of time, for example, for 10 to 14 days, whereby only hybridomas resulting from the cell fusion of the spleen cells and the myeloma cells can be selected.

After the cell fusion, the cells are incubated in a HAT medium for a predetermined period of time, for example, for 10 to 14 days, the supernatant of the culture is collected, and the cells in the wells that have been recognized to be reactive with any of the synthetic peptides mentioned above by ELISA are selected and incubated in an HT medium (or a medium comprising hypoxanthine and thymidine) to thereby scale up the cells. Then, the resulting cells are cloned by a limiting dilution-culture method using non-immunized mouse spleen cells as feeder cells.

After the cloning, these are again subjected to screening by ELISA, whereby clones with high antibody-producing ability which can grow well and are stable are selected to obtain anti-Fas-specific antibody-producing hybridomas. After having been scaled up, the hybridomas are frozen and stored.

To produce a monoclonal antibody according to the present invention, employable is an *in-vitro* method where the hybridomas are incubated in a serum-added medium or a serum-free medium, or an *in-vivo* method where the hybridomas are implanted in the abdomen of a mouse and the ascites is collected from the mice. The culture obtained in the former or the ascites obtained in the latter can be purified to obtain the intended monoclonal antibody according to salting-out with ammonium sulfate, ion-exchange column chromatography, molecular sieve column chromatography, protein A column chromatography, antigen-fixed column chromatography or the like, in the same manner as above.

Both the polyclonal antibody and the monoclonal antibody thus produced can specifically recognize the intracellular region of Fas and, in addition, can also specifically recognize a soluble form Fas containing an intracellular region. The antibodies of this type have not been known at all and are novel physiologically-active substances.

Since the intracellular Fas-specific antibodies of the present invention are novel and have excellent specificity as mentioned hereinabove, these can be utilized for the purification of Fas and can also be utilized for the measurement of the intracellular region of Fas and the soluble form Fas containing an intracellular region. We, the present inventors have further studied in order to simply and accurately carry out the intended measurement and, as a result, have succeeded in the development of a sandwich ELISA method for the measurement.

According to the sandwich ELISA method of the present invention, for example, an antibody that recognizes the extracellular region of Fas is fixed in the solid phase of a microtiter plate while the specific antibody of the present invention is labeled with enzyme such as HRP or the like to form a conjugate, and OPD is used as the substrate. As the standard, utilized was a recombinant Fas (the whole molecule except the inside of the transmembrane region) obtained in a baculovirus expression system. In this manner, we, the present inventors have hereby established an immunoassay system according to such sandwich ELISA.

To the intracellular FAS-specific antibody-labeled conjugate to be used in this immunoassay system, suitably applicable is a technique of forming labeled antibodies. For example, for enzyme labeling, a thiol group is introduced into the specific antibody of the present invention, which is reacted with an enzyme into which a maleimido group has been introduced. Alternatively, the specific antibody of the present invention is digested into F(ab)' fractions and then reacted with an enzyme into which a maleimido group has been introduced. In this manner, the intended labeled antibodies can be obtained.

As the labeling enzymes, for example, suitably usable are ordinary enzymes, such as horseradish peroxidase (HRP) as well as alkali phosphatase (AP), beta-galactosidase, ALB, GOD, etc. Apart from the labeling with such enzymes, also employable in the present invention are fluorescent dyes such as rhodamine, fluorescen isothiocyanate (FITC) and the like and also ferritin for directly labeling the antibodies.

By constructing the immunoassay system according to such sandwich ELISA, it has become possible to accurately and simply measure the intracellular region of a Fas antigen and also a soluble form Fas containing an intracellular region. Referring to its examples mentioned hereinunder, the present invention is described in more detail.

### Example 1: Formation of Antibody

An anti-Fas polyclonal antibody was formed according to the process mentioned below.

### (1) Synthesis of Peptide:

The C-terminal (intracellular region) of a Fas antigen was synthesized, using a peptide synthesizer (Model 431A Peptide Synthesizer), to obtain a synthetic peptide having the peptide sequence (20mer) mentioned below.
Cys-Asn-Ile-Thr-Ser-Asp-Ser-Gln-Asn-Ser-Asn-Phe-Arg-Asn-Glu-Ile-Gln-Ser-Leu-Val

### (2) Formation of Antigen Conjugate:

Using 4 mg of the synthetic peptide obtained in the above and 2 mg of a maleimide-activated KLH (ex Pierce), formed was a peptide-KLH conjugate. Again using 4 mg of the synthetic peptide and 2 mg of a maleimide-activated BSA (ex Pierce), also formed was a synthetic peptide-BSA conjugate.

### (3) Immunization:

Using the synthetic peptide-KLH as an immunogen and using a Freund's complete adjuvant (FCA) or Freund's incomplete adjuvant (FIA), rabbits (Japanese white rabbits) were immunized with the immunogen in the manner mentioned below.
- First Immunization:: Peptide-KLH 0.5 mg + FCA s.c.
- Second to Sixth Immunization:: Peptide-KLH 0.5 mg + FIA s.c.

### (4) Measurement of Titer of Antibody by ELISA:

Using the following three antigens and using the anti-Fas polyclonal antibody as the first antibody and a biotin anti-rabbit IgG (ex Vector) as the second antibody, the titer of the anti-Fas antibody (which means the reactivity of the antibody) was measured according to ELISA.
- Synthetic Peptide-KLH:: 10 µg/ml; 50 µl coating
- Synthetic Peptide-BSA:: 10 µg/ml; 50 µl coating
- Synthetic Peptide :: 10 µg/ml; 50 µl coating
Left at room temperature overnight.

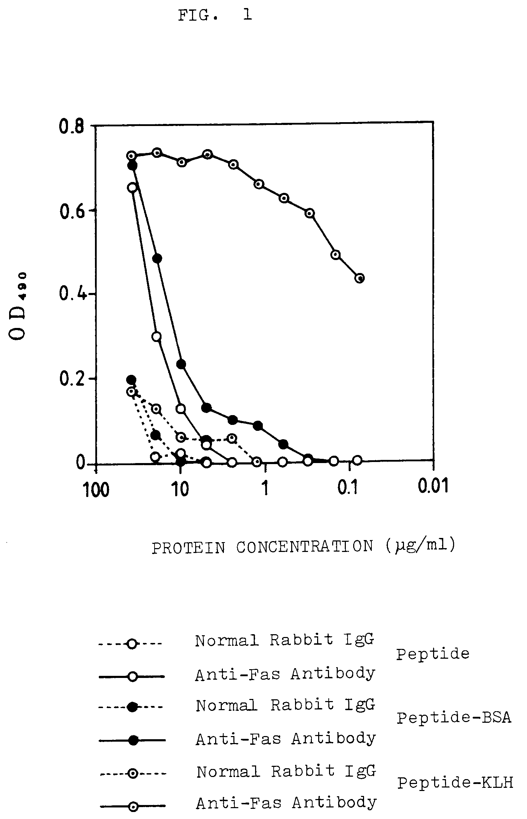

The details of the ELISA conducted for the above are as follows:
Blocking: 1/4 Dilution with Block Ace (ex Dai-Nippon Pharmaceutical), retention time 2 hours
First Antibody: Anti-Fas antibody, retention time 2 hours, washed four times in PBS
Second Antibody: Biotin anti-rabbit IgG (ex Vector), retention time 1 hour, washed four times in PBS
Avidin-biotinated HRP Composite (ex Vector), retention time 30 minutes, washed four times in PBS
OPD (4 mg/10 ml citric acid-phosphoric acid buffer, pH 5.0) + 30 % H₂O₂, 4 µl; 100 µl/well, for about 10 minutes
Stopping: 2 N H₂SO₄, 50 µl/well
Measurement: A490/655
The results obtained are shown in Fig. 1.

### (5) Purification:

The whole blood was collected from each immunized rabbits and then centrifuged at 3000 rpm for 5 minutes to separate the serum. The thus-obtained serum was processed according to the following two-step process to obtain a purified antibody.

### First Step:

By adding 50 ml of saturated ammonium sulfate(SAS) to 50 ml of the serum, a precipitate of gamma-globulin (antibody) was formed. This was centrifuged at 8000 rpm for 15 minutes and then subjected to dialysis in PBS.

### Second Step:

To 50 ml of the antibody thus obtained in the above added was 100 ml of 0.06 M acetate (pH 4.0), and 4 ml of caprylic acid was dropwise added thereto. Next, this was centrifuged at 8000 rpm for 15 minutes. 140 ml of SAS was added to 140 ml of the resulting supernatant, which was then centrifuged at 8000 rpm for 15 minutes. The resulting residue was dissolved in 50 ml of PBS and then subjected to dialysis in PBS in the same manner as in the first step to thereby remove ammonium ions. Thus, a purified antibody was obtained.

### Example 2: Construction of Sandwich ELISA System

A 96-well plate of SUMILON Multi-well Plate (ex Sumitomo Bakelite) for ELISA MS-8496F was used. To each well of the plate added was 50 µl/well of DX-3. (DX-3 is a commercial product available from DNAX Research Institute of Molecular and Cellular Biology, Inc. This is an anti-human Fas monoclonal antibody to be obtained by using, as the immunogen, mouse mastocyte strain P815 that has been transformed to be able to express a membrane-bonded human Fas due to genetic introduction. This antibody recognizes the extracellular region of a Fas antigen. In this example, this antibody DX-3 was diluted to 1/100 with PBS prior to using it herein.) Then, the plate was left at 37°C for 3 hours (or at 4°C overnight).

Next, the supernatant was removed from each well, and 200 µl/well of BLOCK ACE (ex Dai-Nippon Pharmaceuticals; this was diluted into 1/2 with PBS prior to using it herein) was added to each well, which was then kept at 37°C for 2 hours (or at 4°C overnight). Thus, the extracellular Fas-specific antibody was fixed to the micro-titer plate as a solid phase.

After the supernatant was again removed from each well, 50 µl/well of a sample to be tested was added to each well, then kept at 37°C for 2 hours (or at 4°C overnight) and thereafter washed five times with 0.05 % Tween PBS. To this was added 50 µl/well of the antibody (10 µg/ml) that had been obtained in Example 1 and kept at 37°C for 1 hour.

Each well was washed five times with 0.05 % Tween PBS, and then 50 µl/well of a second HRP-labeled antibody (anti-rabbit IgG antibody) was added thereto and kept at 37°C for 1 hour.

This was washed five times with 0.05 % Tween PBS and then two times with PBS. Next, 100 µl/well of OPD/citric acid-phosphoric acid buffer and 1 µl/well of H₂O₂ (1 mg/ml) were added to each well, which was then left as it was for 10 minutes. Then, 100 µl/well of 2 N H₂SO₄ was added thereto, and the OD₄₉₀ was measured. The citric acid-phosphoric acid buffer (pH 5.0) used herein had the following composition.
- 0.1 M Phosphoric Acid·H₂O (21.0 g/liter):: 48.6 ml
- 0.2 M Na₂HPO₄·12H₂O (72.628 g/liter):: 51.4 ml
- DDW:: 100 ml

As the standard, used was a recombinant Fas (the whole molecule except the inside of the cell wall) that had been obtained by incubating baculoviruses. Briefly, the recombinant Fas was produced according to the method mentioned below.

The cDNA of a soluble human Fas (s-h-Fas) was cloned by PCR. From the resulting clone was obtained a recombinant s-h-Fas in an expression system of COS cell/SR-alpha-296 vector or insect-derived Sf-9 cell/baculovirus.

Western blotting of the supernatants of the both cultures with DX2 revealed the production and secretion of s-h-Fas's having a molecular weight of 42 kDa and 35 kDa in the supernatant of the former culture and that of s-h-Fas having a molecular weight of 35 kDa in the supernatant of the latter culture. (DX2 used herein is an anti-human Fas monoclonal antibody that recognizes a different epitope and recognizes the extracellular region of a Fas antigen. This is obtained by immunizing a mouse with mouse mastocyte strain P815 that has been transformed to be able to express a membrane-bonded human Fas due to genetic introduction. DX2 is available also from DNAX.)

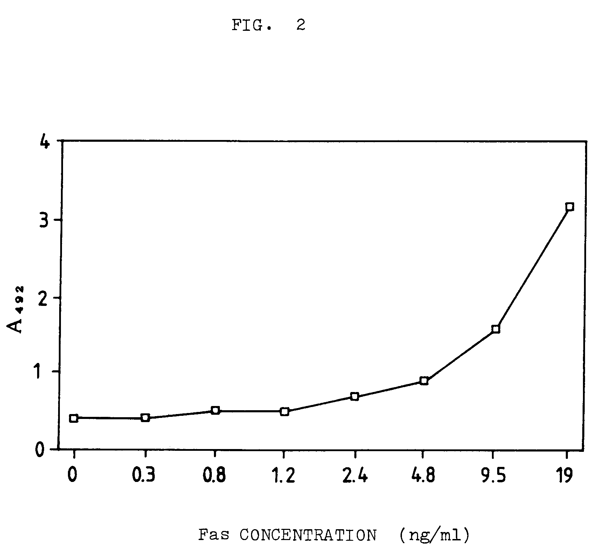

As a result of the measurement of a soluble form Fas according to the method of the present invention, the increase in A₄₉₂ depending on the concentration of the Fas was confirmed (see Fig. 2). Thus, a sandwich ELISA assay system has hereby been established.

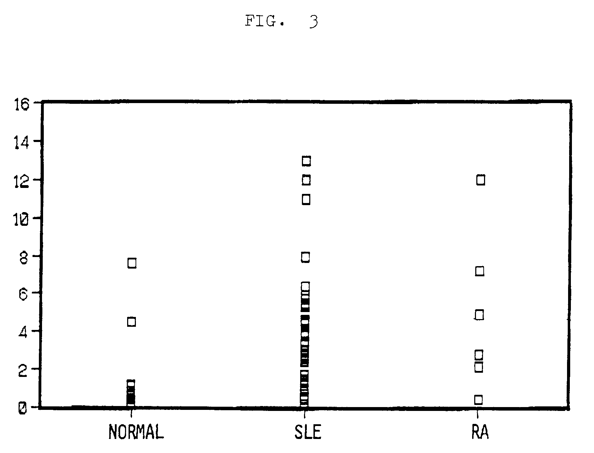

In addition, according to the method of the present invention, the quantitative determination of the s-h-Fas in the sera from SLE patients and RA patients can also be effected simply and accurately. Specifically, it has been confirmed that the s-h-Fas value in SLE patients and RA patients is higher than that in normal persons (see Fig. 3). Thus, it has been verified that the method of the present invention is useful for diagnosis of not only SLE but also RA and other autoimmune diseases.

The preparations of DX-3, DX-2 and the standard s-h-Fas above-mentioned are commercially available from Oriental Yeast Co., Ltd.

### Advantages of the Invention:

According to the present invention, a monoclonal antibody and a polyclonal antibody that specifically recognize the intracellular region of a Fas antigen have been formed for the first time. These antibodies are novel substances that have heretofore been unknown.

The advantages of these antibodies are such that these can recognize not only the intracellular region of a Fas antigen, which could not heretofore been recognized by any conventional antibody, but also a soluble form Fas containing an intracellular region. Thus, the present invention has realized, for the first time, an assay system for a Fas antigen containing an intracellular region.

As has been mentioned hereinabove, it has been found most recently that the serum derived from an SLE patient contains a soluble form Fas. The present invention has made it possible, for the first time, to measure the intracellular region of a Fas antigen. Using the present invention, therefore, it has become possible, for the first time, to clarify the mode of the existence of the soluble form Fas in a patient suffering from an autoimmune disease such as an SLE patient or the like, which, however, has heretofore been not clarified, as to whether it is an extracellular region-derived one or an intracellular region-derived one. Accordingly, on the basis of the difference between the two, exact diagnosis of autoimmune diseases has been realized.

Furthermore, it has heretofore been confirmed that SLE patients have a high value of the soluble form Fas but the value of the soluble form Fas in RA patients has heretofore been considered to be almost comparable to that in normal persons. However, as is obvious from the foregoing description, it has been confirmed according to the method of the present invention that the value of the soluble form Fas in RA patients is higher than that in normal persons. Therefore, the method of the present invention is also extremely useful for the diagnosis of RA.

In addition, the present invention is useful for studying the autoimmunological mechanism in organ transplantation. Further, since the mechanism of the injury to Fas protein-expressed cells is similar to apotosis which is a kind of cell death, the present invention is also useful for studying apotosis.

Moreover, the present invention may be applicable to the development of an assay kit with which sandwich ELISA can be conducted efficiently.

## Claims

1. An antibody that recognizes the intracellular region of a Fas antigen.

2. The antibody as claimed in claim 1, wherein the intracellular region is a C-terminal of a Fas antigen.

3. An antibody to be obtained by the use of a peptide having the following sequence, as the immunogen.
Cys-Asn-Ile-Thr-Ser-Asp-Ser-Gln-Asn-Ser-Asn-Phe-Arg-Asn-Glu-Ile-Gln-Ser-Leu-Val

4. The antibody as claimed in claim 3, wherein a peptide to be obtained by modifying or deleting a part of the sequence is used as the immunogen.

5. The antibody as claimed in any one of claims 1 to 4, which is a polyclonal antibody.

6. The antibody as claimed in any one of claims 1 to 4, which is a monoclonal antibody.

7. A method for producing an antibody as set forth in any one of claims 1 to 5, wherein an animal is immunized with an immunogen of a peptide having the following sequence or a soluble Fas antigen containing an intracellular region, optionally along with a carrier and/or an adjuvant, and the anti-serum thus obtained from the immunized animal is purified.
Cys-Asn-Ile-Thr-Ser-Asp-Ser-Gln-Asn-Ser-Asn-Phe-Arg-Asn-Glu-Ile-Gln-Ser-Leu-Val

8. The method for producing an antibody as claimed in claim 7, wherein the anti-serum is purified by affinity chromatography using a peptide having the following sequence or a peptide to be obtained by modifying or deleting a part of the sequence.
Cys-Asn-Ile-Thr-Ser-Asp-Ser-Gln-Asn-Ser-Asn-Phe-Arg-Asn-Glu-Ile-Gln-Ser-Leu-Val

9. A method for producing an antibody as set forth in any one of claims 1 to 4 or claim 6, wherein an animal is immunized with an immunogen of a peptide having the following sequence or a soluble Fas antigen containing an intracellular region, optionally along with a carrier and/or an adjuvant, the immunized cells obtained from the thus-immunized animal are fused with growable cells to form hybridomas, and the resulting hybridomas are incubated.
Cys-Asn-Ile-Thr-Ser-Asp-Ser-Gln-Asn-Ser-Asn-Phe-Arg-Asn-Glu-Ile-Gln-Ser-Leu-Val

10. The method for producing an antibody as claimed in claim 7 or 9, wherein a peptide to be obtained by modifying or deleting a part of the sequence is used as the immunogen.

11. A method for quantitatively determining a Fas antigen by sandwich ELISA, by using an antibody as set forth in any one of claims 1 to 6 and an antibody that recognizes the extracellular region of a Fas antigen.

12. The method for quantitatively determining a Fas antigen as claimed in claim 11, wherein the Fas antigen is a soluble form Fas antigen.

13. A method for quantitatively determining a Fas antigen by sandwich ELISA, which includes an antibody as set forth in any one of claims 1 to 6.

14. The method for quantitatively determining a Fas antigen as claimed in claim 13, which is for diagnosis of autoimmune diseases.

15. The method for quantitatively determining a Fas antigen as claimed in claim 14, wherein the autoimmune disease is systemic lupus erythematosus (SLE) or rheumatoid arthritis (RA).

16. The antibody as claimed in any one of claims 1 to 6, which is used for quantitatively determining an Fas antigen and/or a soluble form Fas antigen.


(1) GENERAL INFORMATION:

(i) APPLICANT:
(A) NAME: Oriental Yeast Co., Ltd.
(B) STREET: 6-10, Azusawa 3-chome, Itabashi-ku
(C) CITY: Tokyo
(E) COUNTRY: Japan
(F) POSTAL CODE (ZIP): none

(ii) TITLE OF INVENTION: Method for Quantitative Determination of Fas
Antigen

(iii) NUMBER OF SEQUENCES: 1

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25 (EPO)

(v) CURRENT APPLICATION DATA:
APPLICATION NUMBER: EP 95119179.0
(vi) PRIOR APPLICATION DATA:
(A) APPLICATION NUMBER: JP 6-330485
(B) FILING DATE: 08-DEC-1994

(2) INFORMATION FOR SEQ ID NO: 1:

(i) SEQUENCE CHARACTERISTICS:
(A) LENGTH: 20 amino acids
(B) TYPE: amino acid
(D) TOPOLOGY: linear

(ii) MOLECULE TYPE: peptide

(iii) HYPOTHETICAL: NO

(xi) SEQUENCE DESCRIPTION: SEQ ID NO: 1:

Cys Asn Ile Thr Ser Asp Ser Gln Asn Ser Asn Phe Arg Asn Glu Ile
1               5                   10                  15

Gln Ser Leu Val
            20